**Europäisches Patentamt**

**European Patent Office**

**Office européen des brevets**

(19)

(11) Veröffentlichungsnummer: **0 244 610 B1**

## (12) EUROPÄISCHE PATENTSCHRIFT

(45) Veröffentlichungstag der Patentschrift:
27.02.91 Patentblatt 91/09

(51) Int. Cl.⁵: **A61F 2/36**

(21) Anmeldenummer: **87104195.0**

(22) Anmeldetag: **21.03.87**

(30) Priorität: **03.04.86 CH 1303/86**

(43) Veröffentlichungstag der Anmeldung:
**11.11.87 Patentblatt 87/46**

(45) Bekanntmachung des Hinweises auf die
Patenterteilung:
**27.02.91 Patentblatt 91/09**

(84) Benannte Vertragsstaaten:
**AT BE DE ES FR GB IT NL SE**

(56) Entgegenhaltungen:
**EP-A- 0 175 079**
**EP-A- 0 176 421**
**DE-A- 1 913 190**

(73) Patentinhaber: **GEBRÜDER SULZER AKTIENGESELLSCHAFT**
**Zürcherstrasse 9**
**CH-8401 Winterthur (CH)**

(72) Erfinder: **Deckner, André Georges**
**5, Rue de l'Harmonie**
**F-75015 Paris (FR)**
Erfinder: **Imhof, Martin**
**Schöngrund 10**
**CH-6343 Rotkreuz (CH)**
Erfinder: **Zweymüller, Karl, Prof. Dr.-med.**
**Orthop. Universitätsklinik Garnisonstrasse 13**
**A-1090 Wien (AT)**

(74) Vertreter: **Sparing Röhl Henseler**
**Patentanwälte European Patent Attorneys**
**Rethelstrasse 123**
**D-4000 Düsseldorf 1 (DE)**

(54) **Blattartiger Schaft für die Verankerung einer Hüftgelenksprothese im Femur.**

**Beschreibung**

Die Erfindung betrifft einen blattartigen Schaft für die Verankerung einer Hüftgelenksprothese im Femur, wobei die mediale Schmalseite des Schaftes in einer stetig gekrümmten Kurve an einer Prothesenhals-Abschlussebene endet, und wobei ferner die laterale Schmalseite über eine dachfirstartige proximale Begrenzung des Schaftblattes, in der eine Bohrung zum Einsetzen eines Instrumentes vorhanden ist, in den Prothesenhals übergeht, wobei ferner die proximale Begrenzung lateral vom Prothesenhals teilweise von der Abschlussebene gebildet ist.

Schäfte der vorstehend genannten Art sind bekannt (DE-A-1913190, CH-A-642 252 und CH-A-642 839) ; sie werden vorzugsweise bei zementfreier Verankerung der Gelenkkopfprothese im Femur verwendet, wobei die Fixierung in einem Verklemmen und Verkeilen des Schaftblattes in der Kortikalis des operativ vorbereiteten Femurknochens besteht. Dieses Verklemmen erfolgt dabei vorwiegend im Diaphysenbereich, d.h. im distalen Teil des Schaftes, und erfordert eine sorgfältige Anpassung und Abstimmung von Operationshohlraum und Schaftform bzw. -grösse. Es ist daher unter Umständen erforderlich, den Schaft intraoperativ einige Male aus- und einzusetzen. Die bekannten Schäfte haben daher in ihrer dachfirstartig zulaufenden proximalen Begrenzung eine Bohrung, die senkrecht zu den Blattseiten verläuft. Sie dient als Einsatzpunkt für ein beispielsweise hakenförmiges Auszieh- und Ausschlaginstrument.

Da es das allgemeine Bestreben ist, besonders im Trochanterbereich - wegen des Ansatzes von Muskeln und Bändern - soviel Knochensubstanz wie nur möglich zu erhalten, ergeben sich beim Einsetzen des Ausschlaghakens Schwierigkeiten ; denn ohne zusätzliche Verluste von Knochensubstanz im Trochanterbereich können die Haken in die Bohrungen der eingangs beschriebenen Schäfte sehr oft nicht "eingeführt" werden.

Aus der EP-A-0 176 421 ist eine Schaftkonstruktion bekannt, bei der in eine dachfirstartige, proximale Begrenzung von medial her zur Längsachse des Schaftes senkrecht Nuten eingeschnitten sind ; in diese Nuten kann von medial, d.h. vom Prothesenkopf oder -hals her ein Ausricht- und Ausziehinstrument eingeschoben werden.

Aufgabe der Erfindung ist es, bei einem blattartigen Schaft der eingangs genannten Art das Einsetzen des Ausschlaginstrumentes zu erleichtern und ohne zusätzliche Knochenverluste im Trochanterbereich zu ermöglichen. Diese Aufgabe wird nach der Erfindung dadurch gelöst, dass die proximale Begrenzung des Schaftblattes von der Bohrung parallel zu den anterioren und posterioren Blattseiten in der Mittelebene des Schaftblattes vollständig durchsetzt ist.

In den neuen Schaft kann das Ausschlaginstrument vom Prothesenhals her auf einfache Weise eingesetzt werden, ohne dass die Knochenresektion über das für die Aufnahme des Schaftes notwendige Mass ausgedehnt werden muss. Selbstverständlich bringt die neue Konstruktion für Reoperationen früher implantierter Schäfte die gleichen Vorteile wie beim intraoperativen Anpassen. Dabei kann gegebenenfalls das Ausziehinstrument auch von lateral in die Bohrung eingesetzt werden.

Im folgenden wird die Erfindung anhand eines Ausführungsbeispiel im Zusammenhang mit der Zeichnung näher erläutert.

Fig. 1 zeigt eine Ausführungsform des neuen Schaftes in einer Ansicht anterior/posterior ;

Fig. 2 ist in Richtung der Schaftlängsachse eine Aufsicht auf den Schaft von Fig. 1 von proximal nach distal, wobei schematisch das ihn umgebende Knochengewebe des Femurs angedeutet ist.

Das Schaftblatt 1 des Prothesenschaftes erweitert sich von seinem distalen Ende 2 nach allen Seiten konisch, wobei der bonus symmetrisch zu einer Längsmittelachse 3 ausgebildet ist. Die mediale Schmalseite 4 des Konus geht in einen Bogen über, der in einer Ebene 5 endet, die, senkrecht zur Prothesenhalsachse 6 verlaufend, den Prothesenhals 7 gegen das Schaftblatt 1 abschliesst. Der Prothesenhals 7 endet in einem sich nach aussen konisch verjüngenden Zapfen 8, auf den ein nicht gezeigter kugelförmiger Gelenkkopf aufsteckbar ist.

Lateral vom Prothesenhals 7 bildet die Ebene 5 einen Teil der dachfirstartig ausgebildeten proximalen Begrenzung oder Schulter 10 des Schaftblattes, die nach distal in einen Trochanterflügel 11 übergeht. Von diesem führt, sich konisch verjüngend, die laterale Schmalseite 12 zum distalen Ende 2 des Schaftblattes 1.

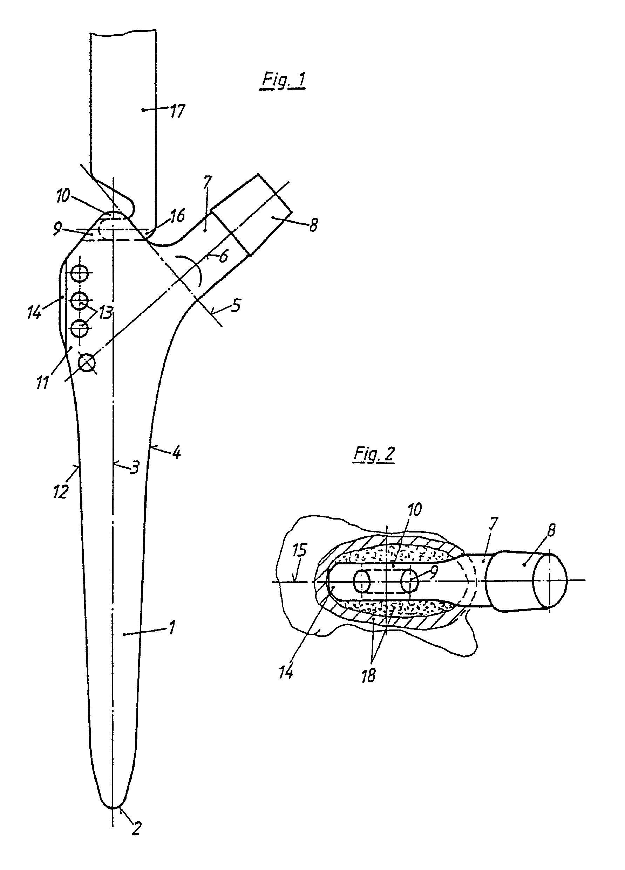

Erfindungsgemäss ist in der proximalen Begrenzung oder Schulter 10 eine Bohrung 9 vorhanden, -die in und parallel zu der Mittelebene 15 (Fig. 2) des Schaftblattes verläuft. Im gezeigten Beispiel erstreckt sich die Bohrung senkrecht zur Längsmittelachse 3 ; sie kann jedoch auch zur Längsmittelachse 3 schiefwinklig von medial nach lateral ansteigend angeordnet sein.

In die Bohrung 9 kann ein Haken 16 eingreifen, der an einem schematisch angedeuteten Ausschlaginstrument 17 sitzt. Dieses dient gleichzeitig zum Führen und Ausrichten des Schaftes beim Einpassen in die Operationsöffnung. In seiner Winkelstellung relativ zur Achse des Instrumentes 17 ist der Haken 16 selbstverständlich an einenrecht- oder schiefwinklingen Verlauf der Bohrung 9 zur Längsmittelachse 3 angepasst.

Das Instrument wird ohne Behinderung - und ohne zusätzliches Entfernen von Knochensubstanz 18 - vom Prothesenhals 7 her in die Bohrung 9 eingeführt. Ein Ausschlagen erfolgt in Richtung der Längsmittelachse 3 bei einem Geradschaft senkrecht nach oben, bei einem gebogenen Schaft entsprechend der Krümmung der Längsmittelachse.

Im Trochanterflügel 11 sind Durchbrüche 13 vorgesehen, die für eine Identifikation der Prothese und/oder für eine Beobachtung der Knochenstruktur im Röntgenbild dienen. Weiterhin weist das äussere Ende des Trochanterflügels 11 eine Verjüngung 14 auf.

## Ansprüche

1. Blattartiger Schaft für die Verankerung einer Hüftgelenksprothese im Femur, wobei die mediale Schmalseite (4) des Schaftes in einer stetig gekrümmten Kurve an einer Prothesenhals-Abschlussebene (5) endet, und wobei die laterale Schmalseite (12) über eine dachfirstartige proximale Begrenzung (10) des Schaftblattes (1), in der eine Bohrung zum Einsetzen eines Instrumentes vorhanden ist, in den Prothesenhals (7) übergeht, wobei ferner die proximale Begrenzung (10) lateral vom Prothesenhals (7) teilweise von der Abschlusseben (5) gebildet ist, dadurch gekennzeichnet, dass die proximale Begrenzung (10) des Schafbkattes (1) von der Bohrung (9) parallel zu den anterioren und posterioren Blattseiten in der Mittelebene (15) des Schaftblattes (1) vollständig durchsetzt ist.

## Claims

1. A blade-like stem for fixing a hip joint prosthesis in the femur, the medial narrow side (4) of the stem terminating in a continuous curve on a closure plane (5) of the prosthesis neck, the lateral narrow side (12) merging into the prosthesis neck (7) by way of a proximal boundary (10) of the stem blade (1), the boundary resembling the ridge of a pitched roof, a bore for the engagement of an instrument being present in the boundary (10), the same being embodied to some extent laterally of the prosthesis neck (7) by the closure plane (5), characterised in that the bore (9) extends completely through the proximal boundary (10) of the stem blade (1) parallel to the anterior and posterior blade sides in the centre-plane (15) of the stem blade (1).

## Revendications

1. Tige en forme de lame pour l'ancrage d'une prothèse d'articulation coxo-fémorale dans le fémur, le côté étroit interne (4) de la tige aboutissant suivant une ligne à courbure continue à un plan de terminaison (5) du col de prothèse, le côté étroit latéral (12) se raccordant au col de prothèse (7) par une délimitation proximale (11), semblable à un faîtage, de la tige en forme de lame (1), dans laquelle est ménagée une perforation servant à l'introduction d'un instrument, et la délimitation proximale (10) étant en outre formée partiellement, sur le côté du col de prothèse (7), par le plan de terminaison (5), caractérisée en ce que la délimitation proximale (11) de la tige en forme de lame (1) est totalement traversée par la perforation (9), parallèlement aux côtés antérieur et postérieur de la lame et dans le plan médian (15) de la tige en forme de lame (1).

_Fig. 1_

_Fig. 2_